(19) Europäisches Patentamt
European Patent Office
Office européen des brevets

(11) **EP 2 547 378 B1**

(12) **EUROPÄISCHE PATENTSCHRIFT**

(45) Veröffentlichungstag und Bekanntmachung des Hinweises auf die Patenterteilung:
**14.08.2019 Patentblatt 2019/33**

(51) Int Cl.:
**A61M 1/16** (2006.01)   **G01F 1/58** (2006.01)
**F16L 9/19** (2006.01)

(21) Anmeldenummer: **11708845.0**

(22) Anmeldetag: **15.03.2011**

(86) Internationale Anmeldenummer:
**PCT/EP2011/053907**

(87) Internationale Veröffentlichungsnummer:
**WO 2011/113838 (22.09.2011 Gazette 2011/38)**

(54) **KASSETTE MIT EINEM SENSOR ZUR BESTIMMUNG DER DIFFERENZ EINES ERSTEN UND EINES ZWEITEN FLÜSSIGKEITSSTROMS**

CARTRIDGE HAVING A SENSOR FOR DETERMINING THE DIFFERENCE BETWEEN A FIRST LIQUID FLOW AND A SECOND LIQUID FLOW

CASSETTE POURVUE D'UN DÉTECTEUR POUR DÉTERMINER LA DIFFÉRENCE D'UN PREMIER ET D'UN DEUXIÈME FLUX DE LIQUIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.04.2010 DE 102010003642**
**15.03.2010 DE 102010002871**

(43) Veröffentlichungstag der Anmeldung:
**23.01.2013 Patentblatt 2013/04**

(60) Teilanmeldung:
**18163445.2 / 3 372 261**

(73) Patentinhaber: **Fresenius Medical Care Deutschland GmbH**
**61352 Bad Homburg v.d.H. (DE)**

(72) Erfinder:
• **NIKOLIC, Dejan**
**60599 Frankfurt (DE)**
• **HEIDE, Alexander**
**65817 Eppstein (DE)**

(74) Vertreter: **Nordmeyer, Philipp Werner**
**df-mp Dörries Frank-Molnia & Pohlman Patentanwälte Rechtsanwälte PartG mbB**
**Theatinerstraße 16**
**80333 München (DE)**

(56) Entgegenhaltungen:
**EP-A2- 0 106 940      DE-A1- 1 951 378**
**DE-A1- 10 313 685      DE-U1-202008 012 801**
**GB-A- 2 003 274      US-A1- 2009 032 232**
**US-A1- 2009 101 550**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung bezieht sich auf eine Kassette zum Fördern eines ersten und eines zweiten Flüssigkeitsstroms, auf eine Vorrichtung zur Dialysebehandlung, und auf eine Verwendung einer entsprechenden Kassette.

Zur Entfernung von harnpflichtigen Substanzen und zum Flüssigkeitsentzug werden beim chronischen Nierenversagen verschiedene Verfahren zur apparativen Blutreinigung bzw. Blutbehandlung eingesetzt. Bei der Hämeodialyse (HD) überwiegt der diffusive Stofftransport, bei der Hämofiltration (HF) liegt ein konvektiver Stofftransport über die Membran vor. Eine Kombination aus beiden Verfahren ist die Hämodiafiltration (HDF). Bei der Peritonealdialyse (PD) wird kein extrakorporaler Kreislauf benötigt und das Peritoneum als Kontaktmembran ausgenutzt.

Wegen der großen Austauschmengen besteht bei den genannten Verfahren, sowie auch bei der kontinuierlichen arterio-venösen HF, der kontinuierlichen veno-venösen HF und der Plasmafiltration (PF) die Notwendigkeit der exakten Bilanzierung von entzogener Flüssigkeit einerseits zur zugeführten Flüssigkeit andererseits und der zu ultrafiltrierenden Menge über die gesamte Behandlungszeit. Zum Stand der Technik gehören gravimetrische und volumetrische Bilanziersysteme.

Darüber hinaus sind auch Verfahren bekannt, die die Flüssigkeitsströme der in den Dialysator einfließenden Flüssigkeiten und der aus dem Dialysator ausfließenden Flüssigkeiten kontinuierlich messen und gegeneinander bilanzieren. Hierzu werden Durchflussmesssensoren verschiedener Ausgestaltungen verwendet.

Die hohe geforderte Genauigkeit und der damit verbundene hohe Aufwand für die Selektion bzw. die Kalibrierung der zur Bilanzierung eingesetzten Durchflusssensoren haben hohe Kosten zur Folge. Darüber hinaus kann es aus Gründen der Handhabbarkeit für das behandelnde medizinische Personal und aus Gründen der Hygiene vorteilhaft sein, möglichst viele den extrakorporalen Blutkreislauf und den Dialysatkreislauf betreffende Vorrichtungen einer Dialysebehandlung in einem Wegwerfdisposable zu integrieren. Wegen des nur einmaligen Gebrauchs dieses Dialysedisposables oder auch Kassette werden hierfür geringe Herstellungskosten angestrebt.

US 2009/0101550 A1 bezieht sich auf ein Dialysesystem, welches eine nicht-invasive Fluidgeschwindigkeitsmessung aufweist. Das Dialysesystem umfasst u.a. eine Kassette zum Fördern eines ersten und eines zweiten Flüssigkeitsstroms, wobei die Kassette eine Vorrichtung zur Bestimmung einer Differenz des ersten und des zweiten Flüssigkeitsstroms und einen ersten Kanal für den ersten Flüssigkeitsstrom und einen zweiten Kanal für den zweiten Flüssigkeitsstrom aufweist.

Aus der DE 103 13 685 A1 ist ein Mikroreaktor bekannt. Der Mikroreaktor umfasst einen Schichtenstapel aus mehreren gleichen Teilen, der mehrere Kanäle ausbildet, wobei in jedem der Teile mehrere Flüssigkeitsläufe auf einer Fläche ausgebildet sind.

Die Aufgabe der vorliegenden Erfindung liegt darin, eine verbesserte Bilanzierung medizinischer Flüssigkeitsströme bereitzustellen.

Die medizinischen Flüssigkeiten sind bevorzugt Dialysat oder Blut.

Die Aufgabe wird gelöst durch eine Kassette zum Fördern eines ersten und eines zweiten Flüssigkeitsstroms in einem Dialysesystem, wobei der erste Flüssigkeitsstrom Dialysatstrom oder Blutstrom sein kann und der zweite Flüssigkeitsstrom Dialysatstrom oder Blutstrom sein kann, wobei die Kassette einen Sensor als Vorrichtung zur Bestimmung der Differenz des ersten und des zweiten Flüssigkeitsstroms aufweist und wobei der Sensor einen ersten Kanal für den ersten Flüssigkeitsstrom und einen zweiten Kanal für den zweiten Flüssigkeitsstrom aufweist.

Die Kassette kann auch zum Fördern von Blut in einem Dialysesystem eingesetzt werden, wobei der erste und der zweite Flüssigkeitsstrom durch den Sensor Blut ist. Denkbar ist auch, dass die Kassette sowohl blutführende, als auch dialysatführende Flüssigkeitssysteme aufweist, wobei der Sensor sowohl Teil des blutführenden Flüssigkeitssystems, als auch des dialysatführenden Flüssigkeitssystems sein kann. In einer alternativen Ausführungsform kann der eine Sensor im ersten Kanal von Blut durchströmt werden und im zweiten Kanal von Dialysat durchströmt werden. In einer ebenfalls alternativen Ausführungsform können zwei der Sensoren jeweils im blutführenden Flüssigkeitssystem als auch im dialysatführenden Flüssigkeitssystem vorhanden sein.

Es ist allgemein anzumerken, dass die vorliegende Erfindung allgemein auf das Bilanzieren von oder Bestimmen der Differenz zwischen zwei Flüssigkeitsströmen, insbesondere medizinischen Flüssigkeitsströmen gerichtet ist. Gemäß einiger Ausführungsbeispiele der vorliegenden Erfindung können diese Flüssigkeitsströme Dialysatströme und/oder Blutströme aufweisen. Wenn im Nachfolgenden ein konkretes Ausführungsbeispiel auf einen konkreten Flüssigkeitsstrom wie Dialysatstrom oder Blutstrom zum Beispiel gerichtet ist, so versteht sich, dass ein ähnliches sinnvolles Ausführungsbeispiel auch auf einen allgemeinen (medizinischen) Flüssigkeitsstrom gerichtet sein kann. Ebenso verhält es sich auch mit Ausführungsbeispielen, die zunächst auf (medizinische) Flüssigkeitsströme allgemein gerichtet sind. Die betreffenden Ausführungsbeispiele können in sinnvoller Weise auch auf konkrete medizinische Flüssigkeitsströme wie z.B. Dialysat- und/oder Blutströme gerichtet sein.

Gemäß der vorliegenden Erfindung wird eine Kassette für ein Dialysesystem bereitgestellt, die einen Sensor als eine kompakte Komponente oder Vorrichtung zur Bilanzierung zweier Flüssigkeitsströme bereitstellt. Mittels der erfindungsgemäßen Kassette wird ein unabhängiges Bilanziersystem ermöglicht, das eine hohe Genauigkeit der Bilanzierung gewährleistet.

Gemäß einem Ausführungsbeispiel der vorliegenden Erfindung sind der erste und der zweite Kanal im Wesentlichen parallel zueinander ausgestaltet; wobei der Sensor ausgestaltet ist, zur Bilanzierung oder Bestimmung der Differenz des ersten und des zweiten Flüssigkeitsstroms den ersten und den zweiten Kanal einem Magnetfeld auszusetzen. Der erste und der zweite Kanal können dabei derart ausgestaltet sein, dass der erste Flüssigkeitsstrom in dem ersten Kanal entgegen dem zweiten Flüssigkeitsstrom in dem zweiten Kanal fließt oder dass der erste und der zweite Flüssigkeitsstrom in die gleiche Richtung fließen.

Gemäß der vorliegenden Erfindung wird der Sensor in Form einer kompakten Komponente oder Vorrichtung zur Bilanzierung zweier Flüssigkeitsströme bereitgestellt, die nur ein Magnetfeld zur Bilanzierung benötigt. Durch die Zusammenführung der beiden Ströme in einem gemeinsamen Magnetfeld werden des Weiteren Fehler in der Bilanzierung der zwei Flüssigkeitsströme deutlich reduziert, die dadurch entstehen könnten, wenn zwei unabhängige Magnetfelder in beispielsweise zwei getrennten Durchflusssensoren von einander abweichen würden. Ferner kann der Sensor als Vorrichtung zur Bilanzierung eines ersten und eines zweiten Flüssigkeitsstroms auf eine kompakte Weise gebaut werden, ohne dass viel Platz zur Unterbringung des Sensors in der Kassette benötigt wird.

Gemäß einem Ausführungsbeispiel der vorliegenden Erfindung entsteht durch das Magnetfeld eine Trennung von positiven und negativen Ladungsträgern in dem ersten Flüssigkeitsstrom des ersten Kanals und in dem zweiten Flüssigkeitsstrom des zweiten Kanals; und der Sensor ist ausgestaltet, eine erste Spannung zu messen, die durch die Trennung von positiven und negativen Ladungsträgern in dem ersten Flüssigkeitsstrom erzeugt wird und durch das Fließen des ersten Flüssigkeitsstroms in dem ersten Kanal entsteht, und eine zweite Spannung zu messen, die durch die Trennung von positiven und negativen Ladungsträgern in dem zweiten Flüssigkeitsstrom erzeugt wird und durch das Fließen des zweiten Flüssigkeitsstroms in dem zweiten Kanal entsteht, wobei die erste und die zweite Spannung zur Bilanzierung oder Bestimmung der Differenz des ersten und des zweiten Flüssigkeitsstroms verwendet werden.

Auf diese Weise erlaubt die vorliegende Erfindung eine sehr genaue Bilanzierung der beiden Flüssigkeits- oder Dialysatströme in einer einzigen Vorrichtung - dem Sensor - unter Verwendung eines einzigen Magnetfeldes auf die Kanäle, durch die die beiden Flüssigkeitsströme fließen.

Gemäß einem Ausführungsbeispiel der vorliegenden Erfindung ist der Sensor ausgestaltet, zumindest ein Signal an eine Vorrichtung zur Dialysebehandlung zu übermitteln, das die gemessene erste und die gemessene zweite Spannung oder einen aus der ersten und zweiten gemessenen Spannung abgeleiteten Wert aufweist. Auf diese Weise wird die Unabhängigkeit der Kassette und des Sensors von anderen Einheiten, Komponenten oder Vorrichtungen eines Dialysesystems gestärkt.

Gemäß einem Ausführungsbeispiel der vorliegenden Erfindung ist an dem ersten Kanal mindestens eine Spannungsmesseinheit des ersten Kanals angebracht, die ausgestaltet ist, die erste Spannung zu messen; und an dem zweiten Kanal ist mindestens eine Spannungsmesseinheit des zweiten Kanals angebracht, die ausgestaltet ist, die zweite Spannung zu messen. Dadurch ist gemäß der vorliegenden Erfindung eine genaue Bestimmung der Spannungen in den Kanälen der Vorrichtung möglich.

Gemäß einem weiteren Ausführungsbeispiel der vorliegenden Erfindung weist der Sensor eine Messzelle auf, die den ersten und den zweiten Kanal aufweist und die dem Magnetfeld derart ausgesetzt wird, dass auf beide Kanäle dieselbe magnetische Feldstärke und derselbe transiente Verlauf des magnetischen Felds wirkt. Dadurch wird eine gleiche Wirkung des Magnetfelds auf die beiden Kanäle in dem Sensor gewährleistet. Die Messzelle kann dabei von dem Magnetfeld umschlossen werden.

Gemäß der vorliegenden Erfindung sind der erste und der zweite Kanal gleich ausgeformt und gleich groß. Insbesondere weisen die Kanäle eine Gleichheit im Bezug auf ihre Form, Größe und Dimension auf. Dadurch wird die Genauigkeit der Bilanzierung unterstützt. Der erste und der zweite Kanal sind aus mindestens drei bezüglich Form und Dimension gleichen Teilen ausgebildet, wobei in jedem der mindestens drei Teile ein Flüssigkeitslauf auf mindestens einer von zwei gegenüberliegenden Flächen ausgeformt ist und wobei die mindestens drei Teile zum Ausbilden des ersten und des zweiten Kanals derart durch ein Auf- oder Anreihen der mindestens drei Teile an den mit Flüssigkeitsläufen ausgeformten Seiten der mindestens drei Teile zusammengesetzt sind, dass zwischen einem ersten und einem zweiten Teil der mindestens drei Teile der erste Kanal und zwischen dem zweiten und einem dritten Teil der mindestens drei Teile der zweite Kanal entsteht, wobei der erste und der zweite Kanal die gleiche Form aufweisen, zueinander parallel sind und nebeneinander oder übereinander angeordnet sind. Gemäß einem Ausführungsbeispiel sind die mindestens drei Teile im Wesentlichen quaderförmig. Gemäß der vorliegenden Erfindung können die mindestens drei Teile auch andere geeignete Formen aufweisen.

Dadurch wird ein äußerst exakter und reproduzierbarer Kanalquerschnitt ermöglicht, der aufgrund der Temperaturabhängigkeit der Kanalgeometrie in Dialysesystemen von großer Wichtigkeit ist. Ferner wird auf diese Weise eine gleiche und sogar identische Ausgestaltung der zwei Kanäle ermöglicht, die für die Genauigkeit der Bilanzierung sorgt und sie darüber hinaus ermöglicht. Gemäß einer Ausgestaltung des vorliegenden Ausführungsbeispiels weisen die mindestens drei Teile Befestigungseinheiten auf, mittels derer die mindestens drei Teile zusammengesteckt werden. Dadurch wird eine schnelle und kostengünstige Produktion der die zwei Kanäle aufweisenden Anordnung ermöglicht, welche der Genauigkeit der Bilanzierung nicht im Wege steht.

Gemäß einer Ausgestaltung des vorliegenden Ausführungsbeispiels sind die mindestens drei Teile mittels eines identischen Spritzwerkzeugs gefertigte Teile. Dadurch wird eine identische oder zumindest im Wesentlichen gleiche Ausgestaltung der Kanäle unterstützt.

Gemäß einer weiteren Ausgestaltung des vorliegenden Ausführungsbeispiels weist der erste Kanal eine weitere Spannungsmesseinheit des ersten Kanals auf, die ausgestaltet ist, eine durch die Trennung erzeugte Spannung in dem ersten Flüssigkeitsstrom zu messen, welche durch Strömen des ersten Flüssigkeitsstroms entsteht; die mindestens eine Spannungsmesseinheit des ersten Kanals ist auf einer Seite des ersten Kanals platziert, die gegenüber einer weiteren Seite des ersten Kanals liegt, auf der die weitere Spannungsmesseinheit des ersten Kanals platziert ist, wobei das Magnetfeld parallel zu der einen und zu der weiteren Seite des ersten Kanals gerichtet ist; der zweite Kanal weist eine weitere Spannungsmesseinheit des zweiten Kanals auf, die ausgestaltet ist, eine durch die Trennung erzeugte Spannung in dem zweiten Flüssigkeitsstrom zu messen, welche durch Strömen des zweiten Flüssigkeitsstroms entsteht; und die mindestens eine Spannungsmesseinheit des zweiten Kanals ist auf einer Seite des zweiten Kanals platziert, die gegenüber einer weiteren Seite des zweiten Kanals liegt, auf der die weitere Spannungsmesseinheit des ersten Kanals platziert ist, wobei das Magnetfeld parallel zu der einen und zu der weiteren Seite des zweiten Kanals gerichtet ist.

Gemäß eines weiteren Ausführungsbeispiels der vorliegenden Erfindung sind der erste und der zweite Kanal durch Einrichten einer Trennwand in einem den ersten und den zweiten Kanal umfassenden Kanal derart ausgeformt, dass der erste und der zweite Kanal eine gleiche Ausformung aufweisen, wobei das Magnetfeld parallel zu der Trennwand gerichtet ist.

Gemäß einer Ausgestaltung des vorliegenden Ausführungsbeispiels ist die mindestens eine Spannungsmesseinheit des ersten Kanals an einer der Trennwand gegenüberliegenden Seite des ersten Kanals platziert; die mindestens eine Spannungsmesseinheit des zweiten Kanals ist an einer der Trennwand gegenüberliegenden Seite des zweiten Kanals platziert; und an der Trennwand ist eine gemeinsame Spannungsmesseinheit angebracht, die ausgestaltet ist, eine durch die Trennung erzeugte Spannung in dem ersten und in dem zweiten Flüssigkeitsstrom zu messen, welche durch Strömen des ersten und des zweiten Flüssigkeitsstroms entsteht, wobei die mindestens eine Spannungsmesseinheit des ersten Kanals, die mindestens eine Spannungsmesseinheit des zweiten Kanals und die gemeinsame Spannungsmesseinheit im Wesentlichen auf gleicher Höhe des ersten und des zweiten Kanals eingerichtet sind.

Gemäß einer anderen Ausgestaltung des vorliegenden Ausführungsbeispiels ist die mindestens eine Spannungsmesseinheit des ersten Kanals an einer der Trennwand gegenüberliegenden Seite des ersten Kanals platziert; die mindestens eine Spannungsmesseinheit des zweiten Kanals ist an einer der Trennwand gegenüberliegenden Seite des zweiten Kanals platziert; und die mindestens eine Spannungsmesseinheit des ersten Kanals und die mindestens eine Spannungsmesseinheit des zweiten Kanals sind im Wesentlichen auf gleicher Höhe des ersten und des zweiten Kanals eingerichtet.

Durch die letzten zwei Ausführungsbeispiele wird deutlich, dass die Kassette und der Sensor als Vorrichtung zum Bilanzieren gemäß der vorliegenden Erfindung sehr variabel und konkreten Bedürfnissen anpassbar ausgestaltet werden können, ohne dass sie ihre oben und nachfolgend genannten Vorteile einbüßen müssen.

Die oben genannte Aufgabe der vorliegenden Erfindung wird auch mittels einer Vorrichtung zur Dialysebehandlung erreicht, wobei die Vorrichtung zur Dialysebehandlung zumindest ein Aufnahmemittel aufweist, das zum Aufnehmen zumindest einer Kassette ausgestaltet ist, die der oben genannten und nachfolgend genauer beschriebenen Kassette entspricht.

Gemäß einem Ausführungsbeispiel ist die Vorrichtung zur Dialysebehandlung ausgestaltet, eine erste Spannung und eine zweite Spannung zu erfassen, wobei die erste Spannung dem ersten Flüssigkeitsstrom und die zweite Spannung dem zweiten Flüssigkeitsstrom entspricht und wobei die erste und die zweite Spannung durch den oben skizzierten und nachfolgend genauer erläuterten Sensor der Kassette gemessen wurden.

Gemäß einem Ausführungsbeispiel der vorliegenden Erfindung weist die Vorrichtung zur Dialysebehandlung eine Steuereinheit auf, die ausgestaltet ist, unter Verwendung des Signals den Fluss einer medizinischen Flüssigkeit zu steuern und/oder zu regeln. Die Steuereinheit ist ausgestaltet, die Förderraten mindestens einer Pumpe zu steuern. Gemäß einem Ausführungsbeispiel der vorliegenden Erfindung weist die Pumpe eine der folgenden Pumpen auf: eine Blutpumpe; eine Dialysatpumpe; eine Ultrafiltrationspumpe.

Die Teile der Kassette werden weiterhin anhand einer nicht beanspruchten Anordnung exemplarisch beschrieben, wonach die mindestens drei bezüglich Form und Dimension gleiche Teile aufweist, wobei in jedem der mindestens drei Teile ein Flüssigkeitslauf auf mindestens einer von zwei gegenüberliegenden Flächen ausgeformt ist und wobei die mindestens drei Teile zum Ausbilden eines ersten und eines zweiten Kanals derart durch ein Auf- oder Anreihen der mindestens drei Teile an den mit Flüssigkeitsläufen ausgeformten Seiten der mindestens drei Teile zusammengesetzt sind, dass zwischen einem ersten und einem zweiten Teil der mindestens drei Teile der erste Kanal und zwischen dem zweiten und einem dritten Teil der mindestens drei Teile der zweite Kanal entsteht, wobei der erste und der zweite Kanal die gleiche Form aufweisen, zueinander parallel sind und nebeneinander oder übereinander angeordnet sind. Gemäß einem Ausführungsbeispiel weisen die mindestens drei Teile eine im Wesentlichen quaderförmige Form auf. Gemäß der vorliegenden Erfindung können die mindestens drei Teile auch andere geeignete Formen aufweisen.

Gemäß einem Beispiel ist die Anordnung zum Einsetzen in eine Vorrichtung zur Bilanzierung eines ersten und eines zweiten Flüssigkeitsstroms ausgestaltet, wobei der erste und der zweite Flüssigkeitsstrom Dialysatströme oder Blutströme sind oder sein können und wobei der erste Kanal für einen ersten Flüssigkeitsstrom und der zweite Kanal für einen zweiten Flüssigkeitsstrom ausgestaltet ist.

Gemäß einem Ausführungsbeispiel der vorliegenden Erfindung entspricht die vorstehend genannte Vorrichtung zur Bilanzierung eines ersten und eines zweiten Flüssigkeitsstroms dem oben skizzierten und nachfolgend genauer erläuterten Sensor der Kassette.

Gemäß einem Ausführungsbeispiel der vorliegenden Erfindung weisen die mindestens drei Teile Befestigungseinheiten auf, mittels derer die mindestens drei Teile zusammengesteckt werden.

Gemäß einem weiteren Ausführungsbeispiel der vorliegenden Erfindung sind die drei Teile mittels eines gleichen Spritzwerkzeugs gefertigte Teile.

Die Kanäle können anhand eines exemplarischen Verfahrens aufgebaut werden, wonach der erste und der zweite Flüssigkeitsstrom Dialysatströme oder Blutströme sind oder sein können, wobei der erste und der zweite Kanal mittels mindestens drei bezüglich Form und Dimension gleichen Teilen aufgebaut werden, wobei in jedem der mindestens drei Teile ein Flüssigkeitslauf auf mindestens einer von zwei gegenüberliegenden Flächen ausgeformt ist und wobei das Verfahren aufweist:

- Zusammensetzen der mindestens drei Teile zum Ausbilden des ersten und des zweiten Kanals durch ein Auf- oder Anreihen der mindestens drei Teile an den mit Flüssigkeitsläufen ausgeformten Seiten der mindestens drei Teile derart, dass zwischen einem ersten und einem zweiten Teil der mindestens drei Teile der erste Kanal und zwischen dem zweiten und einem dritten Teil der mindestens drei Teile der zweite Kanal entsteht, wobei der erste und der zweite Kanal die gleiche Form aufweisen, zueinander parallel sind und nebeneinander oder übereinander angeordnet sind.

Gemäß einem Beispiel weisen die mindestens drei Teile eine im Wesentlichen quaderförmige Form auf. Die mindestens drei Teile können auch andere geeignete Formen aufweisen.

Gemäß einem Beispiel weisen die drei Teile Befestigungseinheiten auf und das Zusammensetzen weist ein Zusammenstecken der drei Teile mittels der Befestigungseinheiten auf.

Die mindestens drei Teile können durch verschiedene Techniken miteinander fluiddicht verbunden werden, z.B. durch Durchlichtlaserverscheißung, Spiegelschweißen, Ultraschallschweißen, Klebung etc.

Durch die oben kurz präsentierten und nachfolgend detaillierter erläuterten Gegenstände der vorliegenden Erfindung wird ein unabhängiges und im Hinblick auf seine Genauigkeit deutlich verbessertes Bilanzieren von Dialysatflüssen ermöglicht. Des Weiteren erlaubt die vorliegende Erfindung eine kostengünstige Lösung zum Bilanzieren von Dialysatflüssen, die dabei auch die hohen Anforderungen an die Genauigkeit der Messungen und an die Präzision der Ausgestaltung und Konstruktion erfüllt.

Im Folgenden werden Ausführungsbeispiele der vorliegenden Erfindung detailliert mit Bezug auf die unten beigefügten Figuren beschrieben.

Es zeigt:

Fig. 1a      das Verwenden einer Kassette zum Fördern von Dialysatströmen in einer Dialysevorrichtung gemäß einem Ausführungsbeispiel der vorliegenden Erfindung;

Fig. 1b      das Verwenden einer Kassette zum Fördern von Dialysatströmen in einer Dialysevorrichtung gemäß einem weiteren Ausführungsbeispiel der vorliegenden Erfindung;

Fig. 2      eine Vorrichtung zur Bilanzierung eines ersten und eines zweiten Flüssigkeitsstroms gemäß einem Ausführungsbeispiel der vorliegenden Erfindung;

Fig. 3a, 3b      in einer Vorrichtung zur Bilanzierung eines ersten und eines zweiten Flüssigkeitsstroms eingerichtete Kanäle für den ersten und den zweiten Flüssigkeitsstroms gemäß einem Ausführungsbeispiel der vorliegenden Erfindung;

Fig. 4a, 4b      in einer Vorrichtung zur Bilanzierung eines ersten und eines zweiten Flüssigkeitsstroms eingerichtete Kanäle für den ersten und den zweiten Flüssigkeitsstroms gemäß zwei weiteren Ausführungsbeispielen der vorliegenden Erfindung;

Fig. 5a, 5b, 5c      eine Anordnung, die zwei Kanäle für zwei Flüssigkeitsströme aufweist, und den Aufbau der Anordnung gemäß einem nicht beanspruchten Beispiel;

Fig. 6a, 6b      eine Anordnung, die zwei Kanäle für zwei Flüssigkeitsströme aufweist, und den Aufbau der Anordnung gemäß einem nicht beanspruchten Beispiel; und

Fig. 7       eine Dialysevorrichtung, die gemäß einem Ausführungsbeispiel der vorliegenden Erfindung ausgestaltet ist.

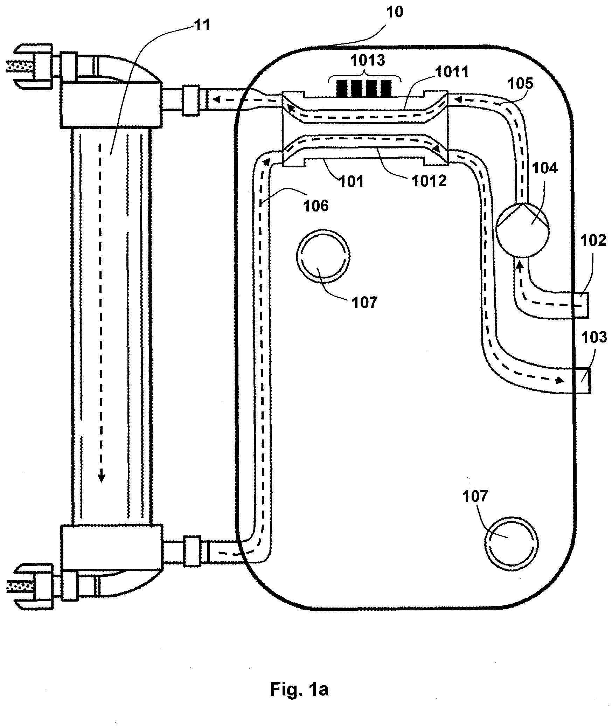

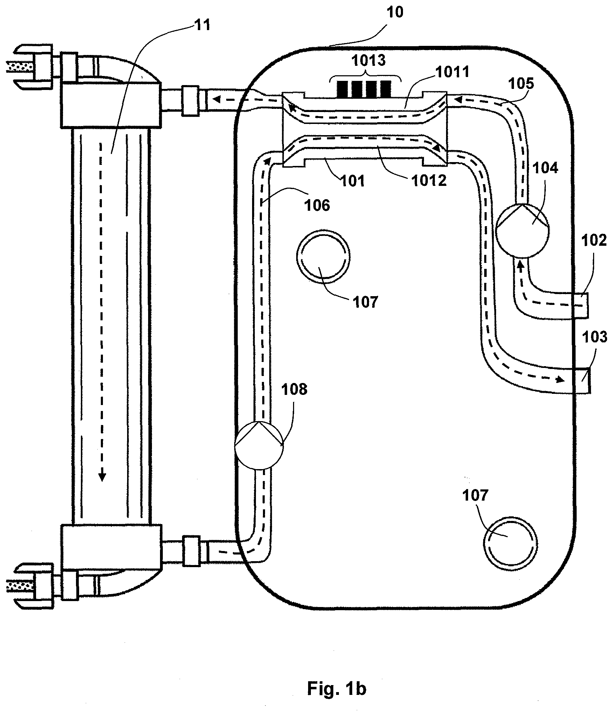

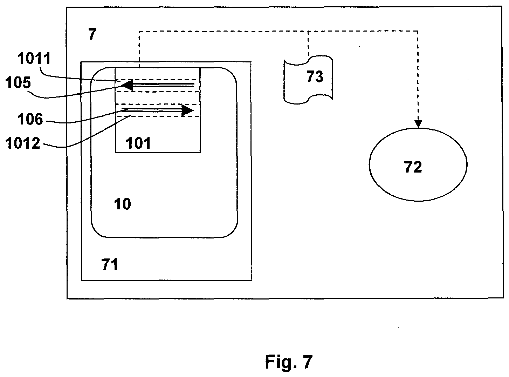

Fig. 1a und Fig. 1b zeigen das Verwenden einer Kassette 10 zum Fördern von Dialysatströmen 105, 106 in einer Dialysevorrichtung gemäß jeweils einem Ausführungsbeispiel der vorliegenden Erfindung. Es ist im Bezug auf Fig. 1a und 1b anzumerken, dass die Einheiten, Komponenten oder Vorrichtungen, die sowohl in Fig. 1a als auch in Fig. 1b die gleichen Bezugszeichen aufweisen, in beiden Ausführungsbeispielen den gleichen Einheiten, Komponenten oder Vorrichtungen entsprechen. Die Dialysevorrichtung ist in Fig. 1a und in Fig. 1b nicht gezeigt, kann aber beispielsweise wie in Fig. 7 angedeutet ausgestaltet sein. Die Dialysevorrichtung weist Aufnahmemittel auf, welche die Kassette 10 aufnehmen können. Die Kassette 10 weist gemäß dem vorliegenden Ausführungsbeispiel zwei Zentrierungseinheiten 107, mittels derer die Kassette 10 in der Dialysevorrichtung in der gewünschten Position arretiert, eingesetzt werden kann. Die Zentrierungen 107 können beispielsweise als Führungslöcher für aus der Dialysevorrichtung ragende Stifte eingeführt werden. Die vorliegende Erfindung erlaubt auch das Verwenden weiterer Mittel zum Einsetzen der Kassette 10 in der Dialysevorrichtung und ist nicht auf die Zentrierungseinheiten 107 beschränkt.

Kassetten zum Fördern von Dialysatströmen wie die Kassette 10 sind allgemein bekannt. Eine herkömmliche Kassette ist beispielsweise in der Patentanmeldung DE 102 24 750 beschrieben. Die in Fig. 1a und in Fig. 1b gezeigten Darstellungen der Kassette 10 richten sich im Wesentlichen auf die für das vorliegende Ausführungsbeispiel notwendigen Teile der erfindungsgemäßen Kassette. Daher sind weitere Vorrichtungen, Einheiten oder Teile eines Dialysekreislaufs (z.B. Sensoren, Aktoren usw.) die sich üblicher Weise in der Kassette 10 befinden können, aus Gründen der Übersichtlichkeit in Fig. 1a und in Fig. 1b nicht gezeigt, -können aber gemäß dem vorliegenden Ausführungsbeispiel in der Kassette 10 enthalten sein.

Die Kassette 10 weist gemäß Fig. 1a und 1b einen Dialysateingang 102 von einer Frischdialysatquellen und einen Dialysatausgang 103 auf.

Zum Pumpen des Dialysatstroms 105 weist die Kassette 10 gemäß dem vorliegenden Ausführungsbeispiel eine Dialysatpumpe 104 auf. Die Förderquote der Dialysatpumpe 104 bestimmt die Höhe der Dialysatflussrate.

Gemäß dem Ausführungsbeispiel der Fig. 1b kann die Kassette 10 überdies eine Ultrafiltrationspumpe 108 aufweisen, die flussabwärts des Dialysefilters 11 Dialysat aus dem Dialysefilter 11 entfernen kann.

Der Dialysatstrom 105 fließt von der Frischdialysatquelle in den Eingang 102 über den Sensor 101, welcher zur Bilanzierung von Flüssigkeitsströmen ausgestaltet ist, zum Dialysefilter 11. Im Dialysefilter 11 findet über die semipermeable Membran des Dialysefilters 11 ein Stoffaustausch zwischen Patientenblut und Dialysat statt, in Folge dessen das Blut des Patienten von Giftstoffen gereinigt wird. Zusätzlich kann durch einen Druckunterschied zwischen Dialysatseite und Blutseite der semipermeablen Membran Flüssigkeit über die Membran aus dem Blut in das Dialysat filtriert werden, um den Patienten zu entwässern. Das Volumen der aus dem Patientenblut auf die Dialysatseite filtrierten Flüssigkeit pro Zeiteinheit wird Ultrafiltrationsrate genannt und wird vom behandelnden Arzt individuell verordnet. Durch den Flüssigkeitsübertritt im Dialysefilter 11 ist der Dialysatstrom 106, der aus dem Dialysatfilter 11 ausfließt, größer als der Dialysatstrom 105, der in den Dialysefilter 11 einfließt. Da die Differenz der beiden Ströme 105, 106 nur aus dem Patientenblut stammen kann, ist die Differenz gleichbedeutend mit der Ultrafiltrationsrate.

Die Ultrafiltrationsrate ist neben den Eigenschaften des Dialysefilters 11 in erster Linie vom Druckunterschied zwischen Blut und Dialysatseite im Dialysefilter 11 abhängig. Es gilt, dass umso mehr Flüssigkeit aus dem Blut auf die Dialysatseite filtriert wird, je höher der Drucküberschuss auf der Blutseite ist. Der sich einstellende Druck auf beiden Seiten der semipermeablen Membran ist proportional zur Förderseite des Blutes und des Dialysats. Somit kann durch eine Steuer- oder Regelung der Förderrate des vorzugsweise Dialysats über die Dialysatpumpe 104 die Ultrafiltrationsrate geregelt werden. Eine Steuerung der Blutpumpe (in Fig. 1a und 1b nicht gezeigt) oder der Blut- und der-Dialysatpumpe ist ebenfalls denkbar.

Da Blut- bzw. Dialysatfluss therapeutisch vom Arzt vorgegeben und damit wenig veränderbare Größen sind, kann der Transmembrandruck auch mit der Ultrafiltrationspumpe 108 erzeugt werden (siehe Fig. 1b). Diese zieht zusätzlich Dialysat 106 flussabwärts des Dialysefilters 11 aus dem Dialysefilter ab. Dies kann kontinuierlich oder periodisch erfolgen. Wird okkludierend flussaufwärts des Dialysators das Dialysat gefördert, z.B. durch die Ausgestaltung der Dialysatpumpe 104 als Schlauchrollenpumpe, muss das von der Ultrafiltrationspumpe 108 zusätzlich aus dem Dialysefilter 11 entfernte Volumen über die semipermeable Membran des Dialysefilters 11 aus dem Blut stammen und stellt somit das Ultrafiltrat dar. Die Ultrafiltration kann in dem Fall kontinuierlich erfolgen. Wird nicht okkludierend Dialysat flussaufwärts des Filters 11 Dialysat gefördert, z.B. durch die Ausgestaltung der Dialysatpumpe 11 als Zentrifugalpumpe, müssen zusätzliche okkludierende Mittel flussaufwärts des Filters 11 vorhanden sein (z.B. Quetschventile, nicht in Fig. 1b gezeigt), die während der Förderung der Ultrafiltrationspumpe 108 das Dialysatflüssigkeitssystem okkludieren. Das von der Ultrafiltrationspumpe 108 zusätzlich entfernte Volumen muss in dem Fall über die semipermeable Membran des Dialysefilters 11 aus dem Blut stammen und stellt somit das Ultrafiltrat dar. Die Ultrafiltration erfolgt dann periodisch. Die Dialysepumpe 104 und die Ultrafiltrationspumpe 108 arbeiten dann abwechselnd.

In beiden Fällen fließt das zusätzliche Flüssigkeitsvolumen, das das Ultrafiltrat ist, über den Sensor 101 und den Kanal 1012. Somit ist das Ultrafiltrationsvolumen bekannt. Die Sensorsignale des Sensors 101 können in weiter unten beschreibender Weise dann auch in dieser Ausführungsform dazu benutzt werden, die Förderrate der Ultrafiltrationspumpe 108 so einzustellen, dass sich die gewünschte Ultrafiltrationsrate einstellt.

Der Sensor 10 weist zum Durchfließen des Blut- oder Dialysatstroms 105, der vom Patienten kommt, einen ersten Kanal 1011 auf und zum Durchfließen des Blut- oder Dialysatstroms 106, der nach dem Filtern zum Patienten fließt, einen zweiten Kanal 1012 auf. Die Bezeichnungen "erster" und "zweiter" sind in dieser Beschreibung nicht als einschränkend zu sehen und dienen lediglich einer besseren Unterscheidung der beiden Kanäle 1011, 1012 des Sensors 10.

Die beiden Kanäle 1011, 1012 sind im Wesentlichen parallel zueinander ausgestaltet. Sie können übereinander oder nebeneinander angeordnet sein, die vorliegende Erfindung weist diesbezüglich keine Einschränkungen auf. Die Blut- oder Dialysatströme 105, 106 fließen im Sensor 10 gemäß dem vorliegenden Ausführungsbeispiel im Gegenstrom aneinander vorbei. Die vorliegende Erfindung ist jedoch nicht auf die gegenströmige Richtung der Ströme 105, 106 beschränkt. Gemäß einem anderen Ausführungsbeispiel können die Flüssigkeitsströme 105, 106 auch in die gleiche Richtung fließen.

Ein weiteres Ausführungsbeispiel, das analog dem Ausführungsbeispiel der Fig. 1 ist, wird dadurch realisiert, dass der Sensor 101 zur Bilanzierung von Blut benutzt wird. Dadurch, dass Flüssigkeit bei der Ultrafiltration von der Blut- auf die Dialysatseite filtriert wird, besteht auch die Möglichkeit, auf der Blutseite zu bilanzieren, da die Blutströme in und aus dem Dialysefilter dann unterschiedlich sind. Diese Differenz kann ebenfalls vom Sensor 101 detektiert werden. Dieses Ausführungsbeispiel kann analog dem Ausführungsbeispiel in Fig. 1 ausgestaltet sein, mit dem Unterschied, dass nun Blut am Eingang 102 über den ersten Kanal 1011 des Sensors 101 fließt. Von dort wird das Blut über den unteren Bluteingang des Dialysefilters durch den Filter und von dem oberen Blutausgang des Filters durch den zweiten Kanal 1012 des Sensors gefördert. Es ist grundsätzlich auch möglich, beide Ausführungen in einer Kassette 10 zu integrieren, die dann zwei Sensoren 101 aufweist.

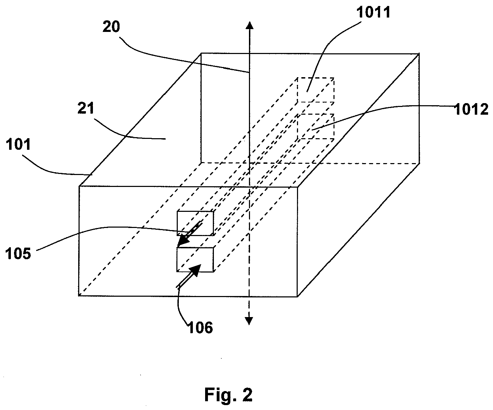

Gemäß dem vorliegenden Ausführungsbeispiel der Fig. 1 erfasst der Sensor 101 die Spannungen, die durch das Strömen des Blut- oder Dialysatstroms 105 im Kanal 1011 und durch das Strömen des Blut- oder Dialysatstroms 106 im Kanal 1012 entstehen. Das Erfassen der Spannungen wird genauer im Bezug auf das Ausführungsbeispiel der Fig. 2 erläutert werden. Die Spannungen werden im Sensor 101 mittels entsprechender Spannungsmesseinheiten erfasst, welche z.B. Elektroden sein können. Das Einsetzen von Spannungsmesseinheiten in diesem Zusammenhang ist im Allgemeinen bekannt und wird deswegen nicht im Einzelnen erläutert, siehe Beispielsweise die Patentanmeldung GB 2 003 274 A, in der das Einsetzen von Elektroden offenbart wird. Gemäß der vorliegenden Erfindung können beliebige geeignete bekannte Spannungsmesseinheiten verwendet werden.

Der Sensor 101 ist ausgestaltet, die erfassten oder gemessenen Spannungen der Blut- oder Dialysatströme 105, 106 in den Kanälen 1011, 1012 an die Dialysevorrichtung zu übermitteln oder zu senden. Gemäß dem vorliegenden Ausführungsbeispiel weist der Sensor 101 vier Spannungsmesseinheiten auf, die in Fig. 1 nicht gezeigt sind, wobei jeweils zwei von denen an einem der Kanäle 1011, 1012 angebracht sind. Die Anzahl der eingesetzten Spannungsmesseinheiten kann gemäß der vorliegenden Erfindung variieren und ist nicht auf die Verwendung von vier Spannungsmesseinheiten eingeschränkt. Gemäß dem vorliegenden Ausführungsbeispiel werden Elektroden als Spannungsmesseinheiten verwendet. Zum Weiterleiten oder Senden der gemessenen Spannungen an die Dialysevorrichtung weist der Sensor 101 zumindest eine Übermittlungseinheit auf. Gemäß dem vorliegenden Ausführungsbeispiel ist diese Übermittlungseinheit ein Kontakt. Ferner werden gemäß dem vorliegenden Ausführungsbeispiel vier Übermittlungseinheiten 1013 als Kontakte eingesetzt. Die Anzahl der eingesetzten Übermittlungseinheiten 1013 kann gemäß der vorliegenden Erfindung variieren und ist nicht auf die Verwendung von vier Übermittlungseinheiten 1013 eingeschränkt. Gemäß dem vorliegenden Ausführungsbeispiel entspricht die Anzahl der Übermittlungseinheiten 1013 der Anzahl der Spannungsmesseinheiten. Dabei ist jede Elektrode, die als Spannungsmesseinheit verwendet wird, einer Übermittlungseinheit bzw. einem Kontakt 1013 zugeordnet. Die Kontakte 1013 sind die Kontakte der vier Elektroden. Die Kontakte 1013 sind gemäß dem vorliegenden Ausführungsbeispiel an einer Seite des Sensors 101 aus dem Sensor 101 herausgeführt und sind mit der Dialysevorrichtung leitend verbunden. Mittels dieser Kontakte 1013 übermittelt der Sensor 101 die bestimmten oder gemessenen Spannungswerte der Dialysevorrichtung, welche die Spannungswerte aus den Kontakten 1013 ablesen kann.

Die Spannungswerte der Kanäle 1011, 1012 werden zum Bilanzieren der Blut- oder Dialysatströme 105, 106 eingesetzt. Dieses wird ausführlicher im Bezug auf die Fig. 8 erläutert.

Es ist anzumerken, dass die vorliegende Erfindung nicht auf die Verwendung von Kontakten 1013 als Übermittlungseinheiten 1013 eingeschränkt ist. So kann z.B. eine Induktivität, als Empfängerspule ausgeführt, in der Kassette Energie per Induktion aus einem magnetischen Wechselfeld, welches die Dialysemaschine oder -Vorrichtung bereitstellt, erzeugen und in bekannter Weise über Gleichrichtung des sich einstellenden Wechselspannung an der Empfängerspule einer in die Kassette integrierten elektronischen Spannung bereitstellen. Diese elektronische Schaltung kann derart eingerichtet sein, die Spannungswerte der Spannungsmesseinheiten des Sensors 101 zu verarbeiten und daraus ein Signal zu erzeugen, welches kontaktlos, z.B. über Funk oder über Lastmodulation der Empfängerspule zur Dialysemaschine oder-Vorrichtung übertragen wird.

Fig. 2 zeigt eine Vorrichtung 101 zur Bilanzierung eines ersten und eines zweiten Flüssigkeitsstroms gemäß einem Ausführungsbeispiel der vorliegenden Erfindung. Die Vorrichtung 101 entspricht gemäß dem vorliegenden Ausführungsbeispiel dem in Fig. 1 gezeigten Sensor 101 und-zeigt eine detaillierte Ausgestaltung des Sensors 101.

Gemäß dem vorliegenden Ausführungsbeispiel weist der Sensor 101 eine Messzelle 21 auf, welche im Inneren des Sensors 101 eingerichtet ist. Die Messzelle 21 umfasst oder weist auf den Kanal 1011 und den Kanal 1012. Gemäß dem vorliegenden Ausführungsbeispiel sind die Kanäle 1011, 1012 beispielhaft als Quader dargestellt. Es ist jedoch anzumerken, dass die vorliegende Erfindung nicht auf quaderförmige Kanäle beschränkt ist, die vorliegende Erfindung ist auch auf andere für Kanäle verwendbare Formen anwendbar, z.B. auf zylinderförmige Kanäle usw. Im Bezug auf ihre Ausformung sind die beiden Kanäle 1011, 1012 gleich oder identisch. Dabei sind sie insbesondere bezüglich ihrer Form, Größe und Dimensionen gleich oder identisch ausgestaltet.

Gemäß dem vorliegenden Ausführungsbeispiel ist die Messzelle 21 des Sensors 101 so konstruiert oder ausgestaltet, dass die beiden Ströme 105, 106 in zwei getrennten Kanälen 1011, 1012 im Gegenstrom aneinander vorbeifließen. Wird die Messzelle 21 im Sensor 101 in ein Magnetfeld eingebracht, das senkrecht zur Strömungsrichtung der Blut- oder Dialysatströme 105, 106 oder zu den Kanälen 1011, 1012 respektive gerichtet ist, tritt eine Ladungsauftrennung der positiven und negativen Ladungsträger im Fluid auf. Die treibende Kraft ist dabei die Lorentz-Kraft. Die zu den Blut- oder Dialysatströmen 105, 106 oder zu den Kanälen 1011, 1012 senkrechte Ausrichtung des Magnetfeldes ist in Fig. 2 mittels des Pfeils 20 verdeutlicht. Zur Bilanzierung der Blut- oder Dialysatströmen 105, 106 wird die durch die Ladungsauftretung erzeugte Spannung in den Kanälen 1011, 1012 gemessen.

Die Potentialdifferenz hängt dabei von der Entfernung bzw. von der Stärke des Magnetfeldes ab. Gemäß dem vorliegenden Ausführungsbeispiel spielt die Leitfähigkeit des Mediums bei dem Sensor 101 keine ausschlaggebende Rolle. Gemäß dem vorliegenden Ausführungsbeispiel sollte eine Mindest-Leitfähigkeit jedoch gegeben sein. Dabei gilt die folgende Formel:

$$U = B * v * D, \qquad (1)$$

wobei U die Spannung, B die Feldstärke, v die Strömungsgeschwindigkeit und D der Innendurchmesser des Kanals 1011, 1012 sind.

Das strömende Blut oder Dialysat respektive im Kanal 1011 erzeugt ebenso eine Spannung wie das Blut oder Dialysat im Kanal 1012. Würde das Blut oder Dialysat aus dem Kanal 1011 nach dem - Austritt aus der Messzelle 21-wieder direkt in den Eingang des Kanals 1012 strömen, so würden sich die induzierten Spannungen exakt aufheben. D.h. eine Differenz der Spannung des ersten Kanals 1011 und der Spannung des zweiten Kanals 1012 wäre gleich Null. Führt eine Differenzbildung der beiden Spannungen zu einem Wert ungleich Null, muss der Volumenstrom in beiden Kanälen 1011, 1012 ungleich sein. Dieses ist in der Regel in Dialysevorrichtungen der Fall, da der Blut- oder Dialysatstrom 105 aus dem Kanal 1011 nach dem Austritt aus der Messzelle 21 nicht wieder direkt in den Eingang des Kanals 1012 als Blut- oder Dialysatstrom 106 strömt, sondern zunächst seinen Weg zum Filter 11 macht, wo es in Folge der Ultrafiltration zu einer Änderung der Flüssigkeitsströme kommt. Auf dem Gebiet der Dialysesysteme wird eine aus Differenzen der Spannungen der Kanäle 1011, 1012 gebildete Rate als Ultrafiltrationsrate bezeichnet.

Gemäß Formel (1) gilt bei identischen Innendurchmessern D1 = D2 = D der beiden Messkanäle 1011 und 1012 und identischer Feldstärke B, dass Feld B1 = B2 = B in beiden Messkanälen 1011, 1012. Siehe Formel (2):

$$U1 - U2 = v1 - v2, \qquad (2)$$

wobei v1 - v2 dem Differenzfluss zwischen den beiden Kanälen 1011, 1012 entspricht, und gemäß der vorliegenden Ausführungen ist dieser Differenzfluss identisch mit der Ultrafiltrationsrate.

Zur Bestimmung der Ultrafiltrationsrate ist es also gemäß der Formel (2) nicht notwendig, die exakte Dimension der Messkanäle 1011, 1012 oder die Stärke des Magnetfelds zu kennen. Erst wenn eine absolute Messung der Flussraten durch die beiden Kanäle erwünscht wird, müssen D und B bekannt sein.

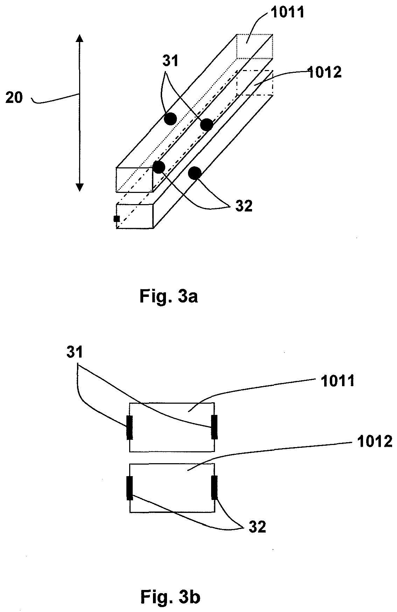

Figuren 3a und 3b zeigen eine detaillierte Sicht auf die Kanäle 1011, 1012 gemäß einem Ausführungsbeispiel der vorliegenden Erfindung. Gemäß dem vorliegenden Ausführungsbeispiel weist sowohl der erste Kanal 1011 als auch der zweite Kanal 1012 Spannungsmesseinheiten zum Messen der Spannungen in den Kanälen 1011, 1012 auf, die wie vorstehend dargelegt durch die Ladungsauftrennung erzeugt werden und durch das Fließen der jeweiligen Flüssigkeitsströme 105, 106 entstehen. Gemäß dem vorliegenden Ausführungsbeispiel sind die Spannungsmesseinheiten 31, 32 Elektroden. Dabei weist jeder Kanal 1011, 1012 an zwei einander gegenüberliegenden Seiten der jeweiligen Kanäle 1011, 1012 je eine Elektrode 31, 32 auf. Gemäß dem vorliegenden Ausführungsbeispiel sind die Elektroden 31, 32 parallel zu der Ausrichtung 20 des Magnetfeldes eingerichtet.

Es ist anzumerken, dass die vorliegende Erfindung nicht auf das Verwenden von zwei Elektroden als Spannungsmesseinheiten pro Kanal 1011, 1012 beschränkt ist. Fig. 3a und Fig. 3b zeigen lediglich eine beispielhafte Ausführungsform der vorliegenden Erfindung.

Da gemäß dem vorliegenden Ausführungsbeispiel zwei Spannungswerte für die Kanäle 1011 und 1012 bereitgestellt werden, wird zum Bilanzieren der Blut- oder Dialyseströme 105, 106 gemäß Formel (2) die Differenz der beiden gemessenen Spannungswerte gebildet. Das anregende Magnetfeld B kann sowohl ein nichtveränderliches Magnetfeld, als auch ein magnetisches Wechselfeld sein. Die sich einstellenden Spannungen sind im Falle eines anregenden nichtveränderlichen Magnetfelds Gleichspannungen, im Falle eines anregenden magnetischen Wechselfeldes Wechselspannungen. In beiden Fällen wird die aktuelle Amplitude der sich einstellenden Spannung vom aktuellen Flüssigkeitsstrom moduliert. Die Differenzbildung der Spannungswerte erfolgt in dem Fachmann bekannter Weise, z.B. über eine analoge Subtraktionsschaltung oder durch Digitalisierung der Messwerte und anschließender mathematischer Operationen in einem dafür vorgesehenen Rechengerät, welches Teil eines Steuergerätes der Dialysemaschine oder-Vorrichtung sein kann.

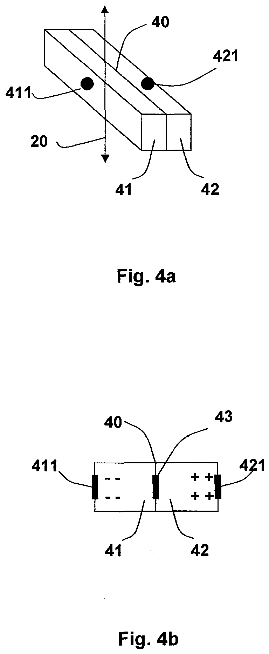

Fig. 4a, 4b zeigen in einer Vorrichtung zur Bilanzierung eines ersten und eines zweiten Flüssigkeitsstroms eingerichtete Kanäle 41, 42 für einen ersten und einen zweiten Flüssigkeits-, Blut- oder Dialysatstrom gemäß zwei weiteren Ausführungsbeispielen der vorliegenden Erfindung.

Gemäß dem Ausführungsbeispiel der Fig. 4a sind die beiden Kanäle 41, 42 durch eine Trennwand 40 getrennt. Dabei ist die Trennwand 40 in einem ursprünglich größeren Kanal derart eingerichtet, dass die Kanäle 41, 42 gleich sind, wobei die Kanäle 41, 42 insbesondere bezüglich ihrer Form, Größe und Dimension gleich sind. Gemäß dem vorliegenden Ausführungsbeispiel weist jeder der Kanäle 41, 42 eine Spannungsmesseinheit 411, 421 auf. Gemäß dem vorliegenden Ausführungsbeispiel sind die Spannungsmesseinheiten 411, 421 Elektroden und sind an den sich gegenüber befindlichen Außenwänden der Kanäle 41, 42 eingerichtet, wobei die Außenwände die parallel zu der Ausrichtung 20 des Magnetfelds gerichteten Außenwände sind. Gemäß dem vorliegenden Ausführungsbeispiel liefert jede Elektrode 411, 421 direkt den Spannungswert des jeweiligen Kanals 41, 42.

Auch gemäß dem Ausführungsbeispiel der Fig. 4b sind die beiden Kanäle 41, 42 durch eine Trennwand 40 getrennt, die wie im Bezug auf Fig. 4a erläutert eingerichtet ist. Gemäß dem Ausführungsbeispiel der Fig. 4b weisen die Kanäle 41, 42 insgesamt drei Spannungsmesseinheiten 411, 421, 43 als Elektroden auf. Das Ausführungsbeispiel der Fig. 4b stellt eine Erweiterung des Ausführungsbeispiels der Fig. 4a dar, wobei eine gemeinsame Spannungsmesseinheit 43 als Elektrode zum Messen der Spannung in beiden Kanälen 41, 42 in der Trennwand 43 zwischen den Elektroden 411, 421 auf der gleichen Höhe zu den Elektroden 411, 421 eingerichtet ist. Ähnlich wie im Ausführungsbeispiel der Fig. 3a und der Fig. 3b respektive, bedarf es hier weiterer Berechnungen, die auf den durch die Elektroden 411, 421, 43 ermittelten Spannungswerte der Kanäle 41, 42 basieren, um den jeweiligen Spannungswert des jeweiligen Kanals 41, 42 zu ermitteln. Wie im Bezug auf Fig. 3a und Fig. 3b erläutert, sind dem Fachmann solche Berechnungen (z.B. anhand Amplituden oder Mittelwerte) geläufig und werden daher nicht in Detail erläutert. Gemäß der vorliegenden Erfindung kann dabei beliebig eine für die jeweilige Situation geeignete geläufige Berechnungsmethode ausgewählt und verwendet werden. Gemäß dem vorliegenden Ausführungsbeispiel wird das Signal symmetrisch um das Potential der gemeinsamen Mittelelektrode gelegt. Dabei wird nur noch eine Auswerteelektronik (welche dem Fachmann geläufig ist) benötigt.

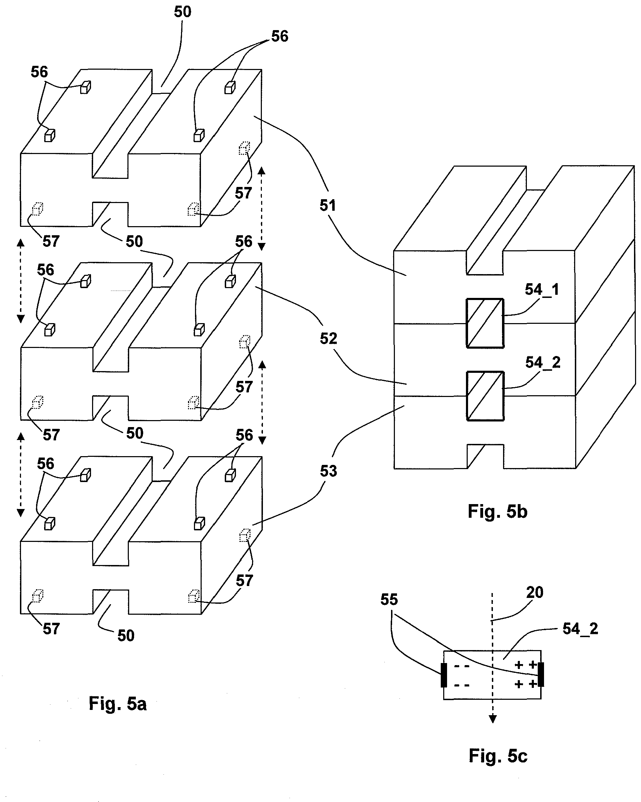

Fig. 5a und Fig. 5b zeigen eine Anordnung, die zwei Kanäle 54_1, 54_2 für zwei Flüssigkeits-, Blut- oder Dialysatströme aufweist, und den Aufbau der Anordnung zur Erläuterung der vorliegenden Erfindung.

Wegen der Temperaturabhängigkeit der Kanalgeometrie ist ein äußerst exakter bzw. reproduzierbarer Kanalquerschnitt notwendig. Diese Forderung steht im Widerspruch zur Forderung eines preiswerten Einwegartikels. Die Kassetten (siehe Kassette 10 der Fig. 1), die in Dialysevorrichtungen zum Fördern von Blut- oder Dialysatströmen respektive eingesetzt werden, repräsentieren Einwegartikel. Aufgrund des Einweg-Charakters der Kassetten ist deren Herstellung möglichst preiswert zu gestalten ist. Gemäß dem vorliegenden Ausführungsbeispiel wird die Messzelle 21 möglichst aus symmetrischen Teilen 51, 52, 53 gefertigt. Gemäß dem vorliegenden Ausführungsbeispiel sind die Teile 51, 52, 53 aus dem gleichen oder einem einzigen Spritzwerkzeug gefertigt. Die drei Teile 51, 52, 53 sind bezüglich ihrer Form und Dimension gleich. Gemäß dem vorliegenden Ausführungsbeispiel sind die Teile 51, 52, 53 im Wesentlichen quaderförmig. Ferner weist jeder der Teile 51, 52, 53 einen Flüssigkeitslauf 50 auf zwei gegenüberliegenden Seiten auf. Die drei Teile 51, 52, 53 werden gemäß dem vorliegenden Ausführungsbeispiel durch eine Aufeinanderreihung der mit einem Flüssigkeitslauf 50 versehenen Seiten zusammengefügt. Gemäß dem vorliegenden Ausführungsbeispiel weisen die Teile 51, 52, 53 auf einer zusammenzufügenden Seite Noppen 56 und auf der anderen zusammenzufügenden Seite zu den Noppen 56 passende Vertiefungen 57 auf, so dass bei dem Aufeinanderreihen der Teile 51, 52, 53 diese mittels der Noppen 56 und der entsprechenden Vertiefungen 57 als Befestigungseinheiten mit einander zusammengesteckt werden. Gemäß dem vorliegenden Ausführungsbeispiel sind die Noppen 56 und die Vertiefungen 57 beispielhaft im Wesentlichen als Quader ausgebildet. Es ist dabei anzumerken, dass die vorliegende Erfindung nicht auf die gezeigte Quaderform der Noppen 56 und Vertiefungen 57 beschränkt ist und dass sie auch weitere geeignete Ausformungen der Noppen 56 und Vertiefungen 57 erlaubt. Ferner können gemäß der vorliegenden Erfindung auch anderweitige geeignete Befestigungseinheiten verwendet werden. Fig. 5b zeigt die fertige, aus den Teilen 51, 52, 53 erstellte Anordnung, die zwei gleiche, zueinander parallele und übereinanderliegende Kanäle 54_1, 54_2 aufweist.

Da jeder Teil 51, 52, 53 einen typischen mechanischen Fehler aufweisen kann, kommt es darauf an, durch geschickte Kombination bzw. Anordnung der Kanäle, jeweils einen oberen Teil-Kanal des oberen Teils 51, 52 und einen unteren Teil-Kanal des entsprechenden unteren Teils 52, 53 zu einem Gesamtkanal 54_1, 54_2 zusammenzufügen. Kleine Vertiefungen bzw. Erhebungen können für die entsprechende Positionierung sorgen. Selbst wenn die Positionierung der Teile 51, 52, 53 nicht exakt ist, wenn also die jeweiligen oberen und unteren Kanalhälften nicht genau übereinander stehen, sind die beiden Gesamtkanäle 54_1, 54_2 identisch.

Auf diese Weise wird mit sehr einfachen Mitteln eine enorme Genauigkeit der Messzelle 21 erreicht.

Gemäß dem vorliegenden Beispiel weisen die Flüssigkeitsläufe 50 eine im Wesentlichen quaderförmige Form auf, was auch zu einer quaderförmigen Ausgestaltung der Gesamtkanäle 54_1, 54_2 führt. Es ist anzumerken, dass diese Ausformung der Flüssigkeitsläufe 50 und der Kanäle 54_1, 54_2 beispielhaft ist und dass gemäß der vorliegenden Erfindung auch weitere geeignete Ausformungen der Flüssigkeitsläufe 50 und folglich der Kanäle 54_1, 54_2 möglich sind.

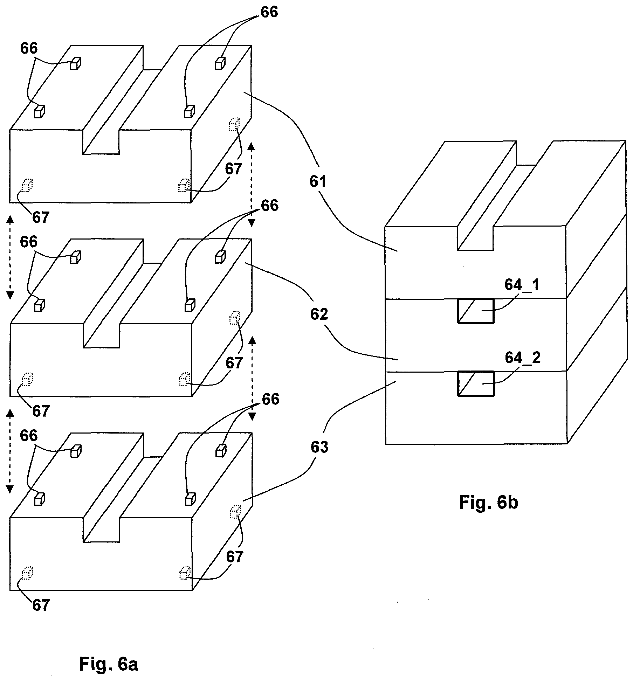

Fig. 6a und Fig. 6b zeigen eine Anordnung, die zwei Kanäle für zwei Flüssigkeits-, Blut- oder Dialysatströme aufweist, und den Aufbau der Anordnung zur Erläuterung der vorliegenden Erfindung.

Das Beispiel der Fig. 6a und Fig. 6b entspricht dem Beispiel der Fig. 5a und der Fig. 5b. Der Unterschied zwischen den beiden Beispielen liegt darin, dass gemäß Fig. 6a und Fig. 6b die Teile 61, 62, 63 nur auf einer Seite jeweils einen Flüssigkeitslauf 60 aufweisen. Das Zusammensetzen der Anordnung gemäß Fig. 6a und Fig. 6b aus den Teilen 61, 62, 63 erfolgt ähnlich dem Zusammensetzen der Teile 51, 52, 53 in Fig. 5a 5b. Gemäß dem vorliegenden Beispiel wird eine mit einem Flüssigkeitslauf 60 ausgestaltete Seite eines Teils 62, 63 mit einer nicht mit einem Flüssigkeitslauf 60 ausgestalteten Seite eines Teils 61, 62 zusammengesetzt. Dabei ist zu beachten, dass die gleichen Teile 61, 62, 63 so zusammengesetzt werden, dass die Flüssigkeitsläufe 60 parallel zueinander und übereinander ausgerichtet sind. Fig. 6b verdeutlicht die durch das Zusammensetzen der Teile 61, 62, 63 entstandenen Kanäle 64_1, 64_2. Der Vorteil dieses Beispiels liegt darin, dass es keiner 100%-igen Positionierung der Teile 61, 62, 63 bedarf und dass geringfügige Abweichungen möglich sind, da kein äußerst sorgfältiges Ausrichten eines oberen Teils des Kanals 54_1, 54_2 zu dem unteren Teil des Kanals 54_1, 54_2 benötigt wird.

Gemäß dem vorliegenden Beispiel weisen die Teile 61, 62, 63 auf einer zusammenzufügenden Seite Noppen 66 und auf der anderen zusammenzufügenden Seite zu den Noppen 66 passende Vertiefungen 67 auf, so dass bei dem Aufeinanderreihen der Teile 61, 62, 63 diese mittels der Noppen 66 und der entsprechenden Vertiefungen 67 als Befestigungseinheiten mit einander zusammengesteckt werden. Ähnlich zu dem Beispiel der Fig. 5a und 5b sind die Noppen 66 und die Vertiefungen 67 des vorliegenden Beispiels beispielhaft im Wesentlichen als Quader ausgebildet. Es ist auch hier anzumerken, dass die vorliegende Erfindung nicht auf die gezeigte Quaderform der Noppen 66 und Vertiefungen 67 beschränkt ist und dass sie auch weitere geeignete Ausformungen der Noppen 66 und Vertiefungen 66 erlaubt, und dass ferner gemäß der vorliegenden Erfindung auch anderweitige geeignete Befestigungseinheiten verwendet werden können.

Gemäß dem Beispiel der Fig. 5a und 5b und gemäß dem Beispiel der Fig. 6a und 6b kann das Magnetfeld senkrecht zu den gestapelten Teilen 51, 52, 53 und 61, 62, 63 ausgerichtet 20 werden. Dieses ist beispielhaft in Fig. 5c dargestellt. Gemäß dem Beispiel der Fig. 5c ist eine Gleichfeldsituation durch Wirken des Magnetfeldes entstanden. Das beispielhaft dargestellte Kanal 54_2 weist zwei Elektroden 55 als Spannungsmesseinheiten auf. Dabei ist die auf der linken Seite angebrachte Elektrode 55 ausgestaltet, die Spannung der negativen Ladungsträger zu messen, und die auf der rechten Seite angebrachte Elektrode 55 ist ausgestaltet, die Spannung der positiven Ladungsträger zu messen. Wie bereits erwähnt, entsteht die Ladungsauftrennung der positiven und negativen Ladungsträger durch das auf den Kanal 54_2 senkrecht 20 wirkende Magnetfeld in der Messzelle 21 oder im Sensor 101 respektive.

Gemäß einem Ausführungsbeispiel der Erfindung kann ein Sensorsignal (das beispielsweise dem Sensor 101 entstammt) als Maß für den Differenzfluss zwischen in eine Dialysevorrichtung einfließendem Dialysat (d.h. Blut- oder Dialysatfluss 105 vom Patienten) und aus der Dialysevorrichtung ausfließendem Dialysat (d.h. Blut- oder Dialysatfluss 106 zum Patienten) dazu verwendet werden, den Fluss der Dialysatpumpe zu regeln.

Mehr Ab- als Zufluss bedeutet dabei, dass Flüssigkeit aus dem Patientenblut über die semipermeable Membran des Dialysefilters in das Dialysat getreten ist. Diese Flüssigkeit pro Zeiteinheit entspricht der Ultrafiltrationsrate.

Die Ultrafiltrationsrate ist therapeutisch vom Arzt vorgeschrieben. Führt man nun das Sensorsignal einer Steuer- oder Regeleinheit zu, die abhängig von dem Ist-Wert des Differenzflusses (Differenz zwischen den Spannungen der Kanäle des Sensors) den Pumpenfluss (d.h. die Einstellungen der Blut- oder Dialysatpumpe) steuert, kann die Ultrafiltrationsrate geregelt werden.

Die Ultrafiltrationsrate ist abhängig vom Transmembranendruck, der im Wesentlichen vom Blut- oder Dialysatfluss 105 vom Patienten und vom Blut- oder Dialysatfluss 106 zum Patienten, sowie den Eigenschaften des Dialysators abhängt (z.B. HighFlux-Eigenschaft).

Durch die Kenntnis des Differenzflusses kann die Pumpenleistung entweder der Dialysatpumpe und/oder der Blutpumpe so angepasst werden, dass sich die gewünschte Ultrafiltrationsrate einstellt. Hierbei wird oft bevorzugt, den Blut- oder Dialysatfluss 105 vom Patienten nicht zu ändern, um den Patienten nicht zu belasten. Ein niedrigerer Blut- oder Dialysatfluss 106 zum Patienten kann einen höheren Transmembrandruck von der Seite des Flusses 105 vom Patienten auf die Seite des Flusses 106 zum Patienten bedeuten, wodurch mehr Flüssigkeit von der Seite des Flusses 105 vom Patienten auf die Seite des Flusses 106 zum Patienten filtriert wird.

Der umgekehrte Fall bedeutet Refiltration, was in der Regel aber nicht erwünscht ist.

Fig. 7 zeigt eine Dialysevorrichtung 7, die gemäß einem Ausführungsbeispiel der vorliegenden Erfindung zur Dialysebehandlung konfiguriert oder ausgestaltet ist. Gemäß dem vorliegenden Ausführungsbeispiel weist die Dialysevorrichtung 7 eine Aufnahmeeinheit 71 auf, welche ausgestaltet ist, zumindest eine Kassette zum Fördern von Blut- oder Dialysatströmen 105, 106 aufzunehmen. Gemäß dem vorliegenden Ausführungsbeispiel handelt es sich bei der Kassette um die im Bezug auf die Fig. 1 beschriebene Kassette 10. In Fig. 7 sind aus Gründen der Übersichtlichkeit im Wesentlichen die für das vorliegende Ausführungsbeispiel relevanten Teile, Einheiten und/oder Komponenten der Dialysevorrichtung 7 dargestellt. Selbstverständlich weist die Dialysevorrichtung 7 auch weitere üblicher Weise in Dialysevorrichtungen enthaltene Teile, Einheiten und/oder Komponenten, welche das bestimmungsgemäße Funktionieren der Dialysevorrichtung 7 ermöglichen. Aus Gründen der Übersichtlichkeit sind diese üblichen Teile, Einheiten und/oder Komponenten einer Dialysevorrichtung in Fig. 7 nicht gezeigt. Wie bereits im Bezug auf das Ausführungsbeispiel der Fig. 1 beschrieben, weist die Kassette 10 den Sensor 101 zur Bilanzierung des Blut- oder Dialysatstroms 105 vom Patienten und des Blut- oder Dialysatstroms 106 zum Patienten auf, wobei die beiden Ströme 105, 106 parallel zueinander und gemäß dem vorliegenden Ausführungsbeispiel in entgegen gesetzten Richtungen in den jeweiligen Kanälen 1011, 1012 des Sensors 101 fließen (die Ströme 105, 106 können auch in die gleiche Richtung fließen).

Gemäß dem vorliegenden Ausführungsbeispiel ist die Dialysevorrichtung 7 konfiguriert oder ausgestaltet, ein Signal zu erfassen, das eine Differenz der im Kanal 1011 und der im Kanal 1012 erfassten Spannung aufweist. Wie bereits vorstehend erläutert, entstehen die jeweiligen Spannungen durch ein senkrechtes Ausrichten 20 eines Magnetfelds auf die Kanäle 1011, 1012 im Sensor 101 und durch das Fließen der jeweiligen Blut- oder Dialysatströme 105, 106 in den Kanälen 1011, 1012. Es ist dabei jedoch anzumerken, dass das senkrechte Ausrichten des Magnetfelds nur ein beispielhaftes Ausrichten des Magnetfeldes darstellt und dass gemäß der vorliegenden Erfindung auch andere Winkel beim Ausrichten des Magnetfeldes zum Tragen kommen können.

Der Sensor 101 weist entsprechende Spannungsmesseinheiten auf, die ausgestaltet sind, die jeweiligen Spannungen in den Kanälen 1011, 1012 zu messen. Die Dialysevorrichtung 8 ist ausgestaltet, die durch die Spannungsmesseinheiten gemessenen Spannungswerte zu erfassen. So zum Beispiel kann dies mittels der in Fig. 1 dargestellten Übermittlungseinheiten 1013, die wie oben dargestellt Kontakte, Spulen oder anderweitige Mittel sein können. Anhand der durch die Spannungsmesseinheiten gemessenen Spannungswerte ermittelt gemäß dem vorliegenden Ausführungsbeispiel die Dialysevorrichtung 7 für jeden Kanal 1011, 1012 den entsprechenden Spannungswert. Wenn mehrere Spannungswerte für einen Kanal 1011, 1012 durch mehrere Spannungsmesseinheiten gemessen, wurden, können hierfür beispielsweise Mittelwertberechnungen verwendet werden. Solche Berechnungen zum Ermitteln eines allgemeinen, repräsentativen Spannungswerts aus mehreren gemessenen Spannungswerten sind dem Fachmann geläufig, daher wird hier nicht detaillierter auf solche Berechnungen eingegangen.

Gemäß dem vorliegenden Ausführungsbeispiel verwendet die Dialysevorrichtung 7 den für den ersten Kanal 1011 ermittelten Spannungswert und den für den zweiten Kanal 1012 ermittelten Spannungswert, um eine Differenz der Spannungen oder Spannungswerte der beiden Kanäle 1011, 1012 zu berechnen. Wie bereits dargelegt, entspricht diese Differenz der Ultrafiltrationsrate.

Gemäß dem vorliegenden Ausführungsbeispiel weist die Dialysevorrichtung 7 eine Steuer- oder Regeleinheit 72 auf, die ausgestaltet ist, mittels der ermittelten Differenz der Spannungen des ersten und des zweiten Kanals 1011, 1012 des Sensors 101 den Fluss zumindest eines der Blut- oder Dialysatströme 105, 106 zu steuern oder zu regeln. Dieses kann die Steuer- oder Regeleinheit 72 zum Beispiel durch ein entsprechendes Einstellen der Blut- und/oder Dialysatpumpe vornehmen, wenn die Steuer- oder Regeleinheit 72 die aktuelle Differenz der Spannungen erhalten hat und nach der Auswertung der Differenz bestimmt hat, dass die Differenz nicht der (vom Arzt) vorbestimmten Ultrafiltrationsrate entspricht. Das Steuern oder Regeln zumindest eines der Blut- oder Dialysatströme 105, 106 wird durch die Steuer- oder Regeleinheit 72 derart vorgenommen, dass die aktuelle Ultrafiltrationsrate oder Differenz der Spannungen in den Kanälen 1011, 1012 nach dem Steuern oder Regeln durch die Steuer- oder Regeleinheit 72 der vorbestimmten Ultrafiltrationsrate oder Differenz der Spannungen in den Kanälen 1011, 1012 entspricht, was z.B. durch ein entsprechendes Einstellen der Blut- und/oder Dialysatpumpe bewirkt werden kann. Gemäß dem vorliegenden Ausführungsbeispiel ist die Dialysevorrichtung 7 nach dem Erfassen der Differenz der aktuellen Spannungen der Kanäle 1011, 1012 bzw. der aktuellen Ultrafiltrationsrate ausgestaltet, ein Signal 73, das die Differenz der aktuellen Spannungen der Kanäle 1011, 1012 bzw. die aktuellen Ultrafiltrationsrate aufweist, an die Steuer- oder Regeleinheit 72 zu übermitteln. Entspricht die Differenz der aktuellen Spannungen der Kanäle 1011, 1012 bzw. die aktuelle Ultrafiltrationsrate nicht der (vom Arzt) vorgegebenen Differenz der aktuellen Spannungen der Kanäle 1011, 1012 bzw. der vorgegebenen Ultrafiltrationsrate, nimmt die Steuer- oder Regeleinheit 72 eine Steuerung oder Regelung der Blut- oder Dialysatströme 105, 106 wie vorstehend beschrieben vor.

Somit bezieht sich die vorliegende Erfindung auf das Bilanzieren von Flüssigkeitsströmen in einem Dialysesystem. Insbesondere bezieht sich die Erfindung auf eine Kassette zum Fördern eines ersten und eines zweiten Flüssigkeitsstroms in einem Dialysesystem, wobei der erste und der zweite Flüssigkeitsstrom medizinische Flüssigkeitsströme wie beispielsweise Dialysatströme oder Blutströme sein können, wobei die Kassette einen Sensor als Vorrichtung zur Bilanzierung des ersten und des zweiten Flüssigkeitsstroms aufweist und wobei der Sensor einen ersten Kanal für den ersten Flüssigkeitsstrom und einen zweiten Kanal für den zweiten Flüssigkeitsstrom aufweist. Ferner bezieht sich die Erfindung auf eine Dialysevorrichtung, die ausgestaltet ist, zumindest eine Kassette aufzunehmen, die wie oben dargelegt ausgestaltet ist.

Obwohl die Erfindung oben mit Bezug auf die Ausführungsbeispielen gemäß der beiliegenden Zeichnungen erklärt wird, ist es ersichtlich, dass die Erfindung nicht auf diese beschränkt ist.

So können zum Beispiel die Kanäle des Sensors oder die Flussläufe der Teile zum Aufbauen einer Anordnung mit zwei Kanälen verschiedene Ausformungen aufweisen oder es können verschiedene Berechnungsmethoden zum Bestimmen eines repräsentativen Spannungswerts für einen Kanal mittels verschiedener für den Kanal gemessener Spannungen oder Spannungswerte respektive verwendet werden.

Bezugszeichenliste

| 10 | Kassette |
| 11 | Dialysefilter |
| 101 | Sensor |
| 102 | Eingang |
| 103 | Ausgang |
| 104 | Dialysatpumpe |
| 105 | Flüssigkeitsstrom |
| 106 | Flüssigkeitsstrom |
| 107 | Zentrierungseinheit |
| 108 | Ultrafiltrationspumpe |
| 1011 | Kanal im Sensor |
| 1012 | Kanal im Sensor |
| 1013 | Übermittlungseinheiten |
| 20 | Ausrichtung des anzuwendenden Magnetfeldes |
| 21 | Messzelle des Sensors |
| 31 | Spannungsmesseinheiten als Elektroden |
| 32 | Spannungsmesseinheiten als Elektroden |
| 40 | Trennwand |
| 41 | Blutstrom, Flüssigkeitsstrom oder Dialysatstrom respektive |
| 42 | Blutstrom, Flüssigkeitsstrom oder Dialysatstrom respektive |
| 411 | Spannungsmesseinheit als Elektrode |
| 421 | Spannungsmesseinheit als Elektrode |
| 43 | Spannungsmesseinheit als Elektrode |
| 50 | Flüssigkeitslauf |
| 51 | erster (Bau-)Teil |
| 52 | zweiter (Bau-)Teil |
| 53 | dritter (Bau-)Teil |
| 54_1 | Kanal |
| 54_2 | Kanal |
| 55 | Spannungsmesseinheit als Elektrode |
| 56 | Noppen als Befestigungseinheiten |
| 57 | Vertiefungen als Befestigungseinheiten |
| 60 | Flüssigkeitslauf |
| 61 | erster (Bau-)Teil |
| 62 | zweiter (Bau-)Teil |
| 63 | dritter (Bau-)Teil |
| 64_1 | Kanal |
| 64_2 | Kanal |
| 66 | Noppen als Befestigungseinheiten |
| 67 | Vertiefungen als Befestigungseinheiten |
| 7 | Dialysevorrichtung |
| 71 | Aufnahmemittel, Aufnahmeeinheit |
| 72 | Regel- oder Steuereinheit |
| 73 | Signal |

**Patentansprüche**

1.  Kassette (10) zum Fördern eines ersten (105) und eines zweiten (106) Flüssigkeitsstroms in einem Dialysesystem, wobei die Kassette einen Sensor (101) als Vorrichtung zur Bestimmung einer Differenz des ersten und des zweiten Flüssigkeitsstroms aufweist und wobei der Sensor einen ersten Kanal (54-1) für den ersten Flüssigkeitsstrom (105) und einen zweiten Kanal (54-2) für den zweiten Flüssigkeitsstrom (106) aufweist, **dadurch gekennzeichnet, dass**

    - der erste (54-1) und der zweite Kanal (54-2) aus mindestens drei bezüglich Form und Dimension gleichen Teilen (51, 52, 53) ausgebildet sind;
    - in jedem der mindestens drei Teile ein Flüssigkeitslauf (50) auf mindestens einer von zwei gegenüberliegenden Flächen ausgeformt ist; und
- die mindestens drei Teile zum Ausbilden des ersten (54-1) und des zweiten Kanals (54-2) derart durch ein Auf- oder Anreihen der mindestens drei Teile (51, 52, 53) an den mit Flüssigkeitsläufen (50) ausgeformten Seiten der mindestens drei Teile (51, 52, 53) zusammengesetzt sind, dass zwischen einem ersten (51) und einem zweiten Teil (52) der mindestens drei Teile (51, 52, 53) der erste Kanal (54-1) und zwischen dem zweiten (52) und einem dritten Teil (53) der mindestens drei Teile (51, 52, 53) der zweite Kanal (54-2) entsteht, wobei der erste (54-1) und der zweite Kanal (54-2) die gleiche Form aufweisen, zueinander parallel sind und nebeneinander oder übereinander angeordnet sind.

2. Kassette (10) nach Anspruch 1, wobei:

   - der erste (54-1) und der zweite Kanal (54-2) im Wesentlichen parallel zueinander ausgestaltet sind; und
   - der Sensor (101) ausgestaltet ist, zur Bestimmung der Differenz des ersten (105) und des zweiten Flüssigkeitsstroms (106) den ersten (54-1) und den zweiten Kanal (54-2) einem Magnetfeld auszusetzen, wobei das Magnetfeld im Wesentlichen senkrecht zu dem ersten (54-1) und zu dem zweiten Kanal (54-2) gerichtet wird.

3. Kassette (10) nach Anspruch 2, wobei:

   - durch das Magnetfeld eine Trennung von positiven und negativen Ladungsträgern in dem ersten Flüssigkeitsstrom (105) des ersten Kanals (54-1) und in dem zweiten Flüssigkeitsstrom (106) des zweiten Kanals (54-2) entsteht; und
   - der Sensor (101) ausgestaltet ist, eine erste Spannung zu messen, die durch die Trennung von positiven und negativen Ladungsträgern in dem ersten Flüssigkeitsstrom erzeugt wird und durch das Fließen des ersten Flüssigkeitsstroms (105) in dem ersten Kanal (54-1) entsteht, und eine zweite Spannung zu messen, die durch die Trennung von positiven und negativen Ladungsträgern in dem zweiten Flüssigkeitsstrom (106) erzeugt wird und durch das Fließen des zweiten Flüssigkeitsstroms (106) in dem zweiten Kanal (54-2) entsteht, wobei die erste und die zweite Spannung zur Bestimmung der Differenz des ersten (105) und des zweiten Flüssigkeitsstroms (106) verwendet werden.

4. Kassette (10) nach Anspruch 3, wobei der Sensor (101) ausgestaltet ist, zumindest ein Signal an ein Dialysesystem zu übermitteln, das die gemessene erste und die gemessene zweite Spannung oder einer aus der ersten und zweiten gemessenen Spannung abgeleiteten Wert aufweist.

5. Kassette (10) nach Anspruch 3 oder 4, wobei:

   - an dem ersten Kanal (54-1) mindestens eine Spannungsmesseinheit (55) des ersten Kanals (54-1) angebracht ist, die ausgestaltet ist, die erste Spannung zu messen; und
   - an dem zweiten Kanal (54-2) mindestens eine Spannungsmesseinheit (55) des zweiten Kanals (54-2) angebracht ist, die ausgestaltet ist, die zweite Spannung zu messen.

6. Kassette (10) nach einem der vorstehenden Ansprüche, wobei die mindestens drei Teile (51, 52, 53) Befestigungseinheiten (56, 57) aufweisen, mittels derer die mindestens drei Teile (51, 52, 53) zusammengesteckt werden.

7. Kassette (10) nach Anspruch 5, wobei

   - der erste Kanal (54-1) eine weitere Spannungsmesseinheit des ersten Kanals (54-1) aufweist, die ausgestaltet ist, eine durch die Trennung erzeugte Spannung in dem ersten Flüssigkeitsstrom (105) zu messen, welche durch Strömen des ersten Flüssigkeitsstroms (105) entsteht;
   - die mindestens eine Spannungsmesseinheit des ersten Kanals (54-1) auf einer Seite des ersten Kanals (54-1) platziert ist, die gegenüber einer weiteren Seite des ersten Kanals (54-1) liegt, auf der die weitere Spannungsmesseinheit des ersten Kanals (54-1) platziert ist, wobei das Magnetfeld parallel zu der einen und zu der weiteren Seite des ersten Kanals (54-1) gerichtet ist;
   - der zweite Kanal (54-2) eine weitere Spannungsmesseinheit des zweiten Kanals (54-2) aufweist, die ausgestaltet ist, eine durch die Trennung erzeugte Spannung in dem zweiten Flüssigkeitsstrom (106) zu messen, welche durch Strömen des zweiten Flüssigkeitsstroms (106) entsteht; und
   - die mindestens eine Spannungsmesseinheit des zweiten Kanals (54-2) auf einer Seite des zweiten Kanals (54-2) platziert ist, die gegenüber einer weiteren Seite des zweiten Kanals (54-2) liegt, auf der die weitere Spannungsmesseinheit des ersten Kanals (54-1) platziert ist, wobei das Magnetfeld parallel zu der einen und zu der weiteren Seite des zweiten Kanals (54-2) gerichtet ist.

8. Vorrichtung (7) zur Dialysebehandlung, umfassend eine Kassette (10) nach einem der Ansprüche 1 bis 7.

**9.** Vorrichtung (7) zur Dialysebehandlung nach Anspruch 8, wobei die Vorrichtung zur Dialysebehandlung (7) ausgestaltet ist, ein Signal (73) zu erfassen, das eine Differenz einer ersten Spannung und einer zweiten Spannung aufweist, wobei die erste Spannung einem ersten Flüssigkeitsstrom (105) und die zweite Spannung einem zweiten Flüssigkeitsstrom (106) entspricht und wobei die erste und die zweite Spannung des Sensors (101) der Kassette (10) gemessen werden.

**10.** Vorrichtung (7) zur Dialysebehandlung nach Anspruch 9, wobei die Vorrichtung eine Steuereinheit (72) aufweist, die ausgestaltet ist, unter Verwendung des Signals (73) Förderraten mindestens einer Pumpe zu steuern, wobei die mindestens eine Pumpe bevorzugt eine der Folgenden ist: eine Blutpumpe; eine Dialysatpumpe; eine Ultrafiltrationspumpe.

**11.** Verwendung einer Kassette (10) gemäß einem der Ansprüche 1 bis 7 in einem Dialysesystem, umfassend:

das Durchströmen des ersten Kanals (54-1) und des zweiten Kanals (54-2) mit einem Dialysatstrom oder einem Blutstrom;
Bestimmen einer Differenz des den ersten Kanal (54-1) durchströmenden ersten Flüssigkeitsstroms und des den zweiten Kanal (54-2) durchströmenden zweiten Flüssigkeitsstroms mittels des Sensors (101).

**12.** Verwendung gemäß Anspruch 11, wobei eine Steuereinheit (72) des Dialysesystems (7) unter Verwendung des mittels des Sensors (101) erzeugten Signals (73) Förderraten mindestens einer Pumpe steuert.

**Claims**

**1.** Cassette (10) for delivering a first fluid stream (105) and a second (106) fluid stream in a dialysis system, the cassette having a sensor (101) as a device for determining a difference between the first and the second fluid stream, and the sensor having a first channel (54-1) for the first fluid stream (105) and a second channel (54-2) for the second fluid stream (106), **characterised in that**

- the first (54-1) and the second channel (54-2) are formed from at least three parts (51, 52, 53) of identical shape and size;
- a fluid course (50) is formed on at least one of two opposite surfaces in each of the at least three parts; and
- to form the first (54-1) and the second channel (54-2), the at least three parts are assembled by the at least three parts (51, 52, 53) being lined up on the sides of the at least three parts (51, 52, 53) formed with fluid courses (50) in such a way that the first channel (54-1) is obtained between a first (51) and a second part (52) of the at least three parts (51, 52, 53) and the second channel (54-2) is obtained between the second (52) and a third part (53) of the at least three parts (51, 52, 53), the first channel (54-1) and the second channel (54-2) having the same shape, being parallel to each other and being arranged alongside each other or one above the other.

**2.** Cassette (10) according to claim 1, wherein:

- the first channel (54-1) and the second channel (54-2) are arranged substantially parallel to each other; and
- the sensor (101) is configured to expose the first (54-1) and the second channel (54-2) to a magnetic field to determine the difference between the first (105) and the second fluid stream (106), the magnetic field being oriented so as to be substantially perpendicular to the first (54-1) and the second channel (54-2).

**3.** Cassette (10) according to claim 2, wherein:

- the magnetic field causes a separation of positive and negative charge carriers in the first fluid stream (105) of the first channel (54-1) and in the second fluid stream (106) of the second channel (54-2); and
- the sensor (101) is configured to measure a first voltage, which is generated by the separation of positive and negative charge carriers in the first fluid stream and is obtained through the flow of the first fluid stream (105) in the first channel (54-1), and to measure a second voltage, which is generated by the separation of positive and negative charge carriers in the second fluid stream (106) and is obtained through the flow of the second fluid stream (106) in the second channel (54-2), the first and the second voltage being used to determine the difference between the first fluid stream (105) and the second fluid stream (106).

**4.** Cassette (10) according to claim 3, wherein the sensor (101) is configured to transmit at least one signal to a dialysis system, said signal having the measured first and the measured second voltage or a value derived from the first and second measured voltage.

**5.** Cassette (10) according to claim 3 or 4, wherein:

- at least one voltage measurement unit (55) of the first channel (54-1) is mounted on the first channel (54-1) and is configured to measure the first voltage; and
- at least one voltage measurement unit (55) of the second channel (54-2) is mounted on the second channel (54-2) and is configured to measure the second voltage.

**6.** Cassette (10) according to any of the preceding claims, wherein the at least three parts (51, 52, 53) have fixing units (56, 57) by means of which the at least three parts (51, 52, 53) are fitted together.

**7.** Cassette (10) according to claim 5, wherein

- the first channel (54-1) has a further voltage measurement unit of the first channel (54-1), which voltage measurement unit is configured to measure a voltage generated by the separation in the first fluid stream (105), which voltage is obtained through the flow of the first fluid stream (105);
- the at least one voltage measurement unit of the first channel (54-1) is placed on a side of the first channel (54-1) opposite another side of the first channel (54-1) on which the further voltage measurement unit of the first channel (54-1) is placed, the magnetic field being oriented parallel to the one side and to the other side of the first channel (54-1);
- the second channel (54-2) has a further voltage measurement unit of the second channel (54-2), which voltage measurement unit is configured to measure a voltage generated by the separation in the second fluid stream (106), which voltage is obtained through the flow of the second fluid stream (106); and
- the at least one voltage measurement unit of the second channel (54-2) is placed on a side of the second channel (54-2) opposite another side of the second channel (54-2) on which the further voltage measurement unit of the first channel (54-1) is placed, the magnetic field being oriented parallel to the one side and to the other side of the second channel (54-2).

**8.** Device (7) for dialysis treatment, comprising a cassette (10) according to any of claims 1 to 7.

**9.** Device (7) for dialysis treatment according to claim 8, the device for dialysis treatment (7) being configured to detect a signal (73) having a difference between a first voltage and a second voltage, the first voltage corresponding to a first fluid stream (105) and the second voltage corresponding to a second fluid stream (106), and the first voltage and the second voltage of the sensor (101) of the cassette (10) being measured.

**10.** Device (7) for dialysis treatment according to claim 9, the device having a control unit (72) configured, by using the signal (73), to control delivery rates of at least one pump, the at least one pump preferably being one of the following: a blood pump; a dialysate pump; an ultrafiltration pump.

**11.** Use of a cassette (10) according to any of claims 1 to 7 in a dialysis system, comprising:

perfusing the first channel (54-1) and the second channel (54-2) with a dialysate stream or a blood stream;
determining a difference between the first fluid stream flowing through the first channel (54-1) and the second fluid stream flowing through the second channel (54-2) using the sensor (101).

**12.** Use according to claim 11, wherein the control unit (72) of the dialysis system (7), by controls delivery rates of at least one pump using the signal (73) generated by means of the sensor (101).

**Revendications**

**1.** Cassette (10) destinée à refouler un premier flux de liquide (105) et un second (106) flux de liquide au sein d'un système de dialyse, dans laquelle la cassette comprend un capteur (101) faisant office de dispositif de détermination d'une différence entre les premier et second flux de liquide et dans laquelle le capteur comprend un premier canal (54-1) destiné au premier flux de liquide (105) et un second canal (54-2) destiné au second flux de liquide (106), **caractérisée en ce que**

- les premier (54-1) et second canaux (54-2) sont formés d'au moins trois parties (51, 52, 53) de forme et dimension identiques ;
- pour chacune des au moins trois parties, une voie de liquide (50) est réalisée sur au moins une des deux surfaces opposées ;
- les au moins trois parties sont rassemblées par alignement ou empilement au niveau des faces, munies des voies de liquide (50), des au moins trois parties (51, 52, 53) afin de former le premier canal (54-1) et le second canal (54-2) de telle manière que le premier canal (54-1) est créé entre une première partie (51) et une deuxième partie (52) parmi les au moins trois parties (51, 52, 53) et le second canal (54-2) est créé entre la deuxième partie (52) et une troisième partie (53) parmi les au moins trois parties (51, 52, 53), dans laquelle le premier canal (54-1) et le second canal (54-2) présentent une forme identique, sont parallèles l'un à l'autre et sont agencés côte à côte ou l'un au-dessus de l'autre.

**2.** Cassette (10) selon la revendication 1, dans laquelle :

- le premier canal (54-1) et le second canal (54-2) sont conçus de manière essentiellement parallèle l'un à l'autre ;
- le capteur (101) est conçu pour exposer le premier canal (54-1) et le second canal (54-2) à un champ magnétique afin de déterminer la différence entre le premier flux de liquide (105) et le second flux de liquide (106), dans laquelle le champ magnétique est essentiellement perpendiculaire au premier canal (54-1) et au second canal (54-2).

**3.** Cassette (10) selon la revendication 2, dans laquelle :

- une séparation des porteurs de charge positifs et négatifs est créée dans le premier flux de liquide (105) du premier canal (54-1) et dans le second flux de liquide (106) du second canal (54-2) grâce au champ magnétique ; et
- le capteur (101) est conçu pour mesurer une première tension qui est produite par la séparation des porteurs de charge positifs et négatifs dans le premier flux de liquide et qui résulte de l'écoulement du premier flux de liquide (105) dans le premier canal (54-1), et pour mesurer une seconde tension qui est produite par la séparation des porteurs de charge positifs et négatifs dans le second courant de liquide (106) et qui résulte de l'écoulement du second courant de liquide (106) dans le second canal (54-2), dans laquelle les première et seconde tensions sont utilisées pour déterminer la différence entre le premier flux de fluide (105) et le second flux de fluide (106).

**4.** Cassette (10) selon la revendication 3, dans laquelle le capteur (101) est conçu pour transmettre à un système de dialyse au moins un signal présentant les première et seconde tensions mesurées ou valeurs dérivées d'une tension mesurée à partir des première et seconde tensions mesurées.

**5.** Cassette (10) selon la revendication 3 ou 4, dans laquelle :

- au moins une unité de mesure de tension (55) du premier canal (54-1) conçue pour mesurer la première tension est installée au niveau du premier canal (54-1) ; et
- au moins une unité de mesure de tension (55) du second canal (54-2) conçue pour mesurer la seconde tension est installée au niveau du second canal (54-2).

**6.** Cassette (10) selon l'une quelconque des revendications précédentes, dans laquelle les au moins trois parties (51, 52, 53) présentent des unités de fixation (56, 57) au moyen desquelles les au moins trois parties (51, 52, 53) sont assemblées.

**7.** Cassette (10) selon la revendication 5, dans laquelle

- le premier canal (54-1) présente une autre unité de mesure de tension du premier canal (54-1) conçue pour mesurer une tension qui est produite par la séparation dans le premier flux de liquide (105) et qui résulte de la circulation du premier flux de liquide (105) ;
- la au moins une unité de mesure de tension du premier canal (54-1) est placée sur un côté du premier canal (54-1) qui se situe à l'opposé d'un autre côté du premier canal (54-1) sur lequel est placée l'autre unité de mesure de tension du premier canal (54-1), dans laquelle le champ magnétique est orienté de manière parallèle à l'un et l'autre côté du premier canal (54-1) ;
- le second canal (54-2) présente une autre unité de mesure de tension du second canal (54-2) conçue pour mesurer une tension qui est produite par la séparation dans le second flux de fluide (106) et qui résulte de la circulation du second flux de fluide (106) ; et
- la au moins une unité de mesure de tension du second canal (54-2) est placée sur un côté du second canal (54-2) qui se situe à l'opposé d'un autre côté du second canal (54-2) sur lequel est placée l'autre unité de mesure de tension du premier canal (54-1), dans laquelle le champ magnétique est orienté de manière parallèle à l'un et l'autre côté du second canal (54-2).

**8.** Dispositif de traitement par dialyse (7), comprenant une cassette (10) selon l'une quelconque des revendications 1 à 7.

**9.** Dispositif de traitement par dialyse (7) selon la revendication 8, dans lequel le dispositif de traitement par dialyse (7) est conçu pour détecter un signal (73) présentant une différence entre une première tension et une seconde tension, dans lequel la première tension correspond à un premier flux de liquide (105) et la seconde tension correspond à un second flux de liquide (106) et dans lequel les première et seconde tensions du capteur (101) de la cassette (10) sont mesurées.

**10.** Dispositif de traitement par dialyse (7) selon la revendication 9, dans lequel le dispositif présente une unité de commande (72) conçue pour commander des débits de refoulement d'au moins une pompe en utilisant le signal (73), dans lequel la au moins une pompe est de manière préférée l'une des pompes ci-après : une pompe à sang ; une pompe à dialysat ; une pompe d'ultrafiltration.

11. Utilisation d'une cassette (10) selon l'une quelconque des revendications 1 à 7 dans un système de dialyse, comprenant les étapes consistant à :

faire traverser le premier canal (54-1) et le second canal (54-2) par un flux de dialysat ou un flux de sang ;
au moyen du capteur (101), déterminer une différence entre le premier flux de liquide traversant le premier canal (54-1) et le second flux de liquide traversant le second canal (54-2).

12. Utilisation selon la revendication 11, dans laquelle une unité de commande (72) du système de dialyse (7) commande des débits de refoulement d'au moins une pompe en utilisant le signal (73) produit au moyen du capteur (101).

**Fig. 1a**

Fig. 1b

**Fig. 2**

**Fig. 3a**

**Fig. 3b**

**Fig. 4a**

**Fig. 4b**

Fig. 5a

Fig. 5b

Fig. 5c

**Fig. 6b**

**Fig. 6a**

**Fig. 7**

**EP 2 547 378 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20090101550 A1
- DE 10313685 A1
- DE 10224750
- GB 2003274 A